(19) Europäisches Patentamt
European Patent Office
Office européen des brevets

(11) EP 2 091 508 B1

(12) **EUROPEAN PATENT SPECIFICATION**

(45) Date of publication and mention of the grant of the patent:
**11.10.2017 Bulletin 2017/41**

(21) Application number: **07867916.4**

(22) Date of filing: **20.12.2007**

(51) Int Cl.:
***A61K 8/39*** *(2006.01)* ***A61K 8/73*** *(2006.01)*
***A61K 8/86*** *(2006.01)* ***A61K 8/898*** *(2006.01)*
***A61Q 5/12*** *(2006.01)*

(86) International application number:
**PCT/US2007/026119**

(87) International publication number:
**WO 2008/079317 (03.07.2008 Gazette 2008/27)**

(54) **PERSONAL CARE COMPOSITION COMPRISING AMINOSILICONE AND NATURALLY DERIVED CATIONIC POLYMERS**

KÖRPERPFLEGEZUSAMMENSETZUNG MIT AMINOSILIKON UND KATIONISCHEN POLYMEREN AUS NATÜRLICHEN QUELLEN

COMPOSITION DE SOINS PERSONNELS COMPRENANT DE L'AMINOSILICONE ET DES POLYMÈRES CATIONIQUES D'ORIGINE NATURELLE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **21.12.2006 US 876243 P**

(43) Date of publication of application:
**26.08.2009 Bulletin 2009/35**

(73) Proprietor: **The Procter & Gamble Company**
**Cincinnati, OH 45202 (US)**

(72) Inventors:
- **PEFFLY, Marjorie Mossman**
  **Cincinnati, OH 45249 (US)**
- **JOHNSON, Eric Scott**
  **Hamilton, OH 45011 (US)**
- **STAUDIGEL, James Anthony**
  **Loveland, OH 45140 (US)**

(74) Representative: **Briatore, Andrea et al**
**Procter & Gamble Service GmbH**
**IP Department**
**Frankfurter Strasse 145**
**61476 Kronberg im Taunus (DE)**

(56) References cited:
**WO-A1-2006/052580 JP-A- 2004 269 556**
**JP-A- 2005 200 308 US-A- 5 346 642**
**US-A1- 2003 224 954 US-A1- 2006 127 345**

- **EDWARDS M ET AL: "CONTROL OF MANNOSE/GALACTOSE RATIO DURING GALACTOMANNAN FORMATION IN DEVELOPING LEGUME SEEDS", PLANTA, SPRINGER VERLAG, DE, vol. 187, no. 1, 1 April 1992 (1992-04-01) , pages 67-74, XP000654833, ISSN: 0032-0935, DOI: DOI:10.1007/BF00201625**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 091 508 B1

## Description

### FIELD OF THE INVENTION

The present invention relates to personal care compositions containing a water-insoluble aminosilicone and naturally derived cationic polymers, which provide a hair conditioning benefit.

### BACKGROUND OF THE INVENTION

Personal care compositions containing various combinations of detersive surfactants and hair conditioning agents are known. These compositions have become more popular among consumers as a means of conveniently obtaining hair conditioning and hair cleansing performance all from a single hair care product.

One approach at improving the overall conditioning performance from a personal care composition involves the use of silicone conditioning agents. These conditioners provide improved hair conditioning performance, as compared to compositions without conditioning agents, and particularly improve the softness and clean feel of dry conditioned hair. These silicone conditioners, however, provide less than optimal deposition of the silicone component to the hair and/or less than optimum conditioning benefits such as dry hair smoothness, hair strand alignment (e.g., minimize frizziness), and ease of combing.

US Patent Application Publication No. 2003/0224954 (Wells et al.) discloses hair conditioning shampoo compositions comprising: a) a detersive surfactant; b) an aminosilicone having a viscosity of from 1,000 cs to 1,000,000 cs, and less than 0.5 wt.% nitrogen; and, c) an aqueous carrier. The compositions may optionally further comprise a non-amino-functionalized silicone having a viscosity of at least about 10,000 cs.

US Patent Application Publication No. 2006/0127345 (Hilvert et al.) discloses shampoo compositions comprising: a) a detersive surfactant; b) silicone particles; c) a protecting agent; and, d) an aqueous carrier. The silicone particles contain at least one amine functional silicone and optionally a non-amine functional silicone.

In order to increase deposition of silicone agents, previous formulations included cationic polymers in combination with the silicone agent. However, consumers have demonstrated a desire for formulations comprising natural ingredients which provide a conditioning benefit. Therefore, there is a need for formulations which incorporate polymers derived from natural sources as deposition aids in combination with silicone conditioning agents.

Additionally, the addition of conditioning agents to personal care compositions can impede cleansing and lathering performance, resulting in heavy hair or insufficient sebum removal. Also, many surfactant systems result in insufficient coacervate formation when combined with deposition polymers.

Based on the foregoing, there is a need for a personal care composition, which provides improved deposition of the silicone component and/or improved hair conditioning benefits using a naturally derived deposition aid. There is also a need for a personal care composition which provides sufficient cleansing, coacervate formation, and lather performance when combined with a natural deposition aid.

### SUMMARY OF THE INVENTION

The present invention is directed to a personal care composition according to the enclosed claims.

### DETAILED DESCRIPTION OF THE INVENTION

While the specification concludes with claims that particularly point out and distinctly claim the invention, it is believed the present invention will be better understood from the following description.

All percentages, parts and ratios are based upon the total weight of the compositions of the present invention, unless otherwise specified. All such weights as they pertain to listed ingredients are based on the active level, and therefore they do not include solvents or byproducts that may be included in commercially available materials, unless otherwise specified. The term "weight percent" may be denoted as "wt.%" herein.

All molecular weights as used herein are weight average molecular weights expressed as grams/mole, unless otherwise specified.

All ratios are weight ratios unless specifically stated otherwise.

Herein, "μ" means microns.

Herein, "cs" means centistoke.

Herein, "molecular weight" is measured in terms of the weight average molecular weight, and is measured by gel permeation chromotography (GPC).

Herein, "graft" means attached to a backbone at any position other than an end group.

Herein, "terminal" means attached to a backbone at an end group.

The term "water-soluble," as used herein, means that the polymer is soluble in water in the present composition. In general, the polymer should be soluble at 25°C at a concentration of at least 0.1% by weight of the water solvent, preferably at least 1%, more preferably at least 5%, most preferably at least 15%.

The term "water-insoluble," as used herein, means that a compound is not soluble in water in the present composition. Thus, the compound is not miscible with water.

The aspects and embodiments of the present invention set forth in this document have many advantages. For example, it has been discovered that aminosilicone deposition is enhanced by addition of the naturally derived cationic polymer described herein. Various embodiments of the present invention further address the need for providing improved hair conditioning benefits, including, e.g., dry hair softness, smoothness, hair strand alignment (i.e., minimization of frizzy hair), ease of dry combing and/or a general conditioned hair feel.

Detersive Surfactant

The personal care composition of the present invention includes a detersive surfactant. The detersive surfactant is included to provide cleaning performance to the composition. The detersive surfactant may be selected from the group consisting of anionic detersive surfactants, zwitterionic or amphoteric surfactants, and combinations thereof. Such surfactants should be physically and chemically compatible with the essential components described herein, or should not otherwise unduly impair product stability, aesthetics or performance.

Suitable anionic detersive surfactants for use in the personal care composition include those which are known for use in hair care or other personal care cleansing compositions. The concentration of the anionic surfactant component in the composition should be sufficient to provide the desired cleaning and lather performance, and generally range from about 5% to about 50%, preferably from about 8% to about 30%, more preferably from about 10% to about 25%, even more preferably from about 12% to about 22%.

Preferred anionic detersive surfactants for use in the compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof.

Suitable amphoteric or zwitterionic detersive surfactants for use in the composition herein include those which are known for use in hair care or other personal care cleansing. Concentrations of such amphoteric detersive surfactants may range from about 0.5% to about 20%, preferably from about 1% to about 10%. Non-limiting examples of suitable zwitterionic or amphoteric surfactants are described in U.S. Patent Nos. 5,104,646 (Bolich Jr. et al.), and 5,106,609 (Bolich Jr. et al.).

In considering the performance characteristics of a personal care composition, such as coacervate formation, wet conditioning performance, dry conditioning performance, and conditioning ingredient deposition on hair, it is necessary to optimize the levels and types of surfactants in order to maximize the performance potential of polymer systems. Specifically, coacervate formation is believed to be essential for efficient deposition of conditioning agents. It has been found that coacervate does not readily form unless the cationic polymers are present in an optimized surfactant system. It is also essential to the practical application of an optimized surfactant system that it comprises practical levels of various surfactants that will allow the overall formulation to meet cost targets. Therefore, the anionic surfactant system for use in the personal care compositions of the present invention has an ethoxylate level and an anion level, wherein the ethoxylate level is from about 1.5 to about 6, and wherein the anion level is from about 1.5 to about 6. The combination of such an anionic surfactant system, with the high charge density cationic naturally derived cationic polymers herein, provide enhanced deposition of conditioning agents to hair and/or skin without reducing cleansing performance.

An optimal ethoxylate level can be calculated by first determining the numbers of ethoxylate moles in a given surfactant molecule. Along with a molecular weight value for the surfactant molecule and the associated stoichiometry of the chemical structure, the total amount of ethoxylate molecules or molar Level of Ethoxylate can thus be calculated. Similarly, a total anion level can be calculated given values for the surfactant molecular weight and values for the amount of anionization reaction completeness. Analytical techniques have been developed to determine values for ethoxylation and anionization within a given surfactant system. The Level of ethoxylate and the Level of Anion (both molar levels) representative of a particular surfactant system are calculated from the percent ethoxylation and percent anion of individual surfactants in the following manner:

**Level of Ethoxylate in a composition** = [% Ethoxylation] * [% Active Ethoxylated Surfactant] *(based upon the total weight of the composition).*

**Level of Anion in a composition** = [% Anion in Ethoxylated Surfactant] * [% Active Ethoxylated Surfactant] *(based upon the total weight of the composition)* + [% Anion in Non-Ethoxylated Surfactant] * [% Active Non-Ethoxylated surfactant] *(based upon the total weight of the composition).*

If a composition comprises two or more surfactants having different respective anions (e.g., surfactant A has a sulfate group and a surfactant B has a sulfonate group), the Level of Anion in the composition is the sum of the molar levels of each respective anion as calculated above.

**Sample Calculation:**

Example 1 shows an ethoxylated surfactant that contains 0.294321 % ethoxylate and 0.188307% sulfate as the anion and a non-ethoxylated surfactant that contains 0.266845% sulfate as an anion. Both surfactants are 29% active.

**Level of Ethoxylate in Example 1** = [0.294321] * [7] (% active ethoxylated surfactant).

Thus, the Level of Ethoxylate in the composition of Example 1 is approximately 2.06.

**Level of Anion in Example 1** = [0.188307] * [7] (% active ethoxylated surfactant) + 0.266845 * [7] (% active non-ethoxylated surfactant).

Thus, the level of Anion in the composition of Example 1 is approximately 3.19.

**Aminosilicone**

The personal care composition of the invention also comprises a water insoluble, non-volatile aminosilicone, which may be one or more polyalkyl siloxanes, one or more polyalkylaryl siloxanes, or mixtures thereof. The aminosilicone is insoluble in the aqueous matrix of the composition and so is present in an emulsified form, with the aminosilicone present as dispersed particles.

Suitable polyalkyl siloxanes include polydimethyl siloxanes which have the CAFTAN designation dimethicone, having a viscosity of from about 5 cs to about 1,000,000 cs, more preferably from about 5,000 cs to about 500,000 cs, and most preferably from about 10,000 cs to about 300,000 cs, each at 25° C. These siloxanes are available commercially from the General Electric Company as the Viscasil series and from Dow Corning as the DC 200 series. Preferred aminosiloxanes include GE Y-14945 and GE Y-14935, each are commercially available from the General Electric Company. The viscosity can be measured by means of a glass capillary viscometer as set out further in Dow Corning Corporate Test Method CTM004 Jul. 20, 1970.

Also suitable is polydiethyl siloxane.

The polyalkylaryl siloxanes which may be used in the compositions of the invention include polymethylphenyl polysiloxanes having a viscosity of from 15 to 65 centistokes at 25° C. The siloxanes are available commercially from the General Electric Company as SF1075 methyl phenyl fluid or from Dow Corning as 556 Cosmetic Grade Fluid.

Also suitable are silicone gums, such as those described in U.S. Pat. No. 4,152,416 (Spitzer), and on General Electric Silicone Rubber product Data Sheet SE 30, SE 33, SE 54 and SE 76. "Silicone gum" denotes polydiorganosiloxanes having a molecular weight of from about 50,000 to about 1,000,000 and specific examples include polydimethyl siloxane polymers, polydimethyl siloxane/diphenyl/methylvinylsiloxane copolymers, polydimethylsiloxane/methylvinylsiloxane copolymers and mixtures thereof.

Herein "aminosilicone" means any amine functionalized silicone; i.e., a silicone containing at least one primary

amine, secondary amine, tertiary amine, or a quaternary ammonium group. Preferred aminosilicones will typically have less than about 0.5% nitrogen by weight of the aminosilicone, more preferably less than about 0.2%, more preferably still, less than about 0.10%. Higher levels of nitrogen (amine functional groups) in the aminosilicone tend to result in both less friction reduction and very low deposition of the aminosilicone to the hair; and consequently, minimal to no conditioning benefit from the aminosilicone component.

In one embodiment, the aminosilicone material may be in the form of a pre-formed emulsion. Herein, the term, "pre-formed emulsion" means an emulsion which is created externally from the personal care composition, which is added only upon formation of the emulsion. If a pre-formed emulsion is included, the average particle size of the aminosilicone material in this emulsion is generally less than or equal to about 30 μ. In one embodiment, the average particle size of the aminosilicone material is less than about 1μ. In yet another embodiment, the average particle size of the aminosilicone material is from about 1 μ to about 30 μ, more preferably from about 2 μ to 30 μ. Particle size is measured by means of a laser light scattering technique, using a 2600D Particle Sizer from Malvern Instruments.

The pre-formed emulsion may be prepared by high shear mechanical mixing of the aminosilicone and water, or by emulsifying the insoluble, non-volatile aminosilicone with water and an emulsifier--mixing the aminosilicone into a heated solution of the emulsifier for instance, or by a combination of mechanical and chemical emulsification. A further suitable technique for preparation of the emulsions is emulsion polymerisation. Emulsion polymerised aminosilicones as such are described in U.S. Pat. No. 2,891,920 (Hyde), U.S. Pat. No. 3,294,725 (Findlay) and U.S. Pat. No. 3,360,491 (Axon).

For non-preformed emulsions, the desired particle size of the aminosilicone in the shampoo composition is formed based on the method of addition which includes controlling the viscosity of the personal care composition prior to addition of the aminosilicone and the mixing speed.

Any surfactant materials either alone or in admixture may be used as emulsifiers in the preparation of the pre-formed silicone emulsions. Suitable emulsifiers include anionic, cationic and nonionic emulsifiers. Examples of anionic emulsifiers are alkylarylsulphonates, e.g., sodium dodecylbenzene sulphonate, alkyl sulphates e.g., sodium, lauryl sulphate, alkyl ether sulphates, e.g., sodium lauryl ether sulphate nEO, where n is from 1 to 20 alkylphenol ether sulphates, e.g., octylphenol ether sulphate nEO where n is from 1 to 20, and sulphosuccinates, e.g., sodium dioctylsulphosuccinate.

Examples of nonionic emulsifiers are alkylphenol ethoxylates, e.g., nonylphenol ethoxylate nEO, where n is from 1 to 50, alcohol ethoxylates, e.g., lauryl alcohol nEO, where n is from 1 to 50, ester ethoxylates, e.g., polyoxyethylene monostearate where the number of oxyethylene units is from 1 to 30.

Typically, a pre-formed emulsion will contain about 25% to about 50% of aminosilicone. Pre-formed emulsions are available from suppliers of aminosilicone oils such as Dow Corning, General Electric, Union Carbide, Wacker Chemie, Shin Etsu, Toshiba, Toyo Beauty Co, and Toray Silicone Co. Examples are the material sold as DC-1310 by Dow Corning, and the materials sold as X-52-1086, X-52-2127 and X-52-2112 by Shin-Etsu.

The compositions of the invention typically contain from 0.01 to 20% by weight, preferably from 0.1 to 10%, more preferably from 0.25 to 3% by weight of insoluble, non-volatile aminosilicone. If less than 0.01% by weight is present in the composition, little conditioning benefit is observed, and if more than 20% by weight is present, the hair will appear greasy.

The aqueous pre-formed emulsion may be incorporated into the personal care composition in an amount of from 0.02 to 40% by weight, preferably from 0.2 to 20% by weight.

The exact quantity of emulsion will of course depend on the concentration of the emulsion, and should be selected to give the desired quantity of insoluble, non-volatile silicone, in the final composition.

**Naturally Derived Cationic Polymer**

The personal care compositions of the present invention comprise a naturally derived cationic polymer. The term, "naturally derived cationic polymer" as used herein, refers to cationic polymers which are obtained from natural sources. The natural sources may be selected from celluloses, starches, galactomannans and other sources found in nature. The naturally derived cationic polymer has a molecular weight from about 1,000 to about 10,000,000, and a cationic charge density at least about 3.0 meq./g, more preferably at least about 3.2 meq/g. Preferably the cationic charge density is also less than about 7 meq/g. The naturally derived polymers are present in an amount of at least 0.05 wt.% of the personal care composition. Preferably, the polymers are present at a range of from about 0.05% to about 10%, and more preferably from about 0.05% to about 5%, by weight of the composition.

The naturally derived cationic polymers aid in deposition of the aminosilicone conditioning agents described herein. Such deposition enhancement results in hair feel, wet conditioning, shine and other appreciable benefits.

The cationic polymers are either soluble in the personal care composition, or preferably are soluble in a complex coacervate phase in the personal care composition formed by the cationic polymer and the anionic detersive surfactant component described hereinbefore. Complex coacervates of the cationic polymer can also be formed with other charged materials in the personal care composition.

Coacervate formation is dependent upon a variety of criteria such as molecular weight, component concentration, and ratio of interacting ionic components, ionic strength (including modification of ionic strength, for example, by addition of salts), charge density of the cationic and anionic components, pH, temperature and the aforementioned surfactant system. Coacervate systems and the effect of these parameters have been described, for example, by J. Caelles, et al., "Anionic and Cationic Compounds in Mixed Systems", Cosmetics & Toiletries, Vol. 106, April 1991, pp 49-54, C. J. van Oss, "Coacervation, Complex Coacervation and Flocculation", J. Dispersion Science and Technology, Vol. 9 (5,6), 1988-89, pp 561-573, and D. J. Burgess, "Practical Analysis of Complex Coacervate Systems", J. of Colloid and Interface Science, Vol. 140, No. 1, November 1990, pp 227-238, which descriptions are incorporated herein by reference.

It is believed to be particularly advantageous for the cationic polymer to be present in the personal care composition in a coacervate phase, or to form a coacervate phase upon application or rinsing of the composition to or from the hair. Complex coacervates are believed to more readily deposit on the hair. Thus, in general, it is preferred that the cationic polymer exist in the personal care composition as a coacervate phase or form a coacervate phase upon dilution. If not already a coacervate in the personal care composition, the cationic polymer will preferably exist in a complex coacervate form in the personal care composition upon dilution with water.

Techniques for analysis of formation of complex coacervates are known in the art. For example, microscopic analyses of the personal care compositions, at any chosen stage of dilution, can be utilized to identify whether a coacervate phase has formed. Such coacervate phase will be identifiable as an additional emulsified phase in the composition. The use of dyes can aid in distinguishing the coacervate phase from other insoluble phases dispersed in the personal care composition.

### Cellulose or Guar Cationic Deposition Polymers

The personal care compositions of the present invention may include cellulose or guar cationic deposition polymers. Such cellulose or guar deposition polymers have a charge density from about 3 meq/g to about 4.0 meq/g at the pH of intended use of the personal care composition, which pH will generally range from about pH 3 to about pH 9, preferably between about pH 4 and about pH 8. The pH of the compositions of the present invention are measured neat.

Suitable cellulose cationic polymers include those which conform to the following formula:

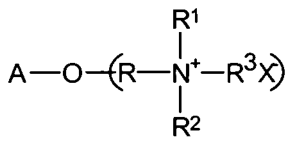

wherein A is an anhydroglucose residual group, such as a cellulose anhydroglucose residual; R is an alkylene oxyalkylene, polyoxyalkylene, or hydroxyalkylene group, or combination thereof; $R^1$, $R^2$, and $R^3$ independently are alkyl, aryl, alkylaryl, arylalkyl, alkoxyalkyl, or alkoxyaryl groups, each group containing up to about 18 carbon atoms, and the total number of carbon atoms for each cationic moiety (*i.e.,* the sum of carbon atoms in $R^1$, $R^2$ and $R^3$) preferably being about 20 or less; and X is an anionic counterion. Non-limiting examples of such counterions include halides (e.g., chlorine, fluorine, bromine, iodine), sulfate and methylsulfate. The degree of cationic substitution in these polysaccharide polymers is typically from about 0.01 to about 1 cationic groups per anhydroglucose unit.

In one embodiment of the invention, the cellulose polymers are salts of hydroxyethyl cellulose reacted with trimethyl ammonium substituted epoxide, referred to in the industry (CTFA) as Polyquaternium 10 and available from Amerchol Corp. (Edison, N.J., USA).

Other suitable cationic deposition polymers include cationic guar gum derivatives, such as guar hydroxypropyltrimonium chloride, specific examples of which include the Jaguar series (preferably Jaguar C-17®) commercially available from Rhone-Poulenc Incorporated.

### Cationically Modified Starch Polymer

The personal care compositions may also comprise a water-soluble cationically modified starch polymer. As used herein, the term "cationically modified starch" refers to a starch to which a cationic group is added prior to degradation of the starch to a smaller molecular weight, or wherein a cationic group is added after modification of the starch to achieve a desired molecular weight. The definition of the term "cationically modified starch" also includes amphoterically modified starch. The term "amphoterically modified starch" refers to a starch hydrolysate to which a cationic group and an anionic group are added.

The cationically modified starch polymers disclosed herein have a percent of bound nitrogen of from about

0.5% to about 4%. The cationically modified starch polymers also have a molecular weight of from about 50,000 to about 10,000,000.

The cationically modified starch polymers have a charge density at least about 3.0 meq/g. The chemical modification to obtain such a charge density includes, but is not limited to, the addition of amino and/or ammonium groups into the starch molecules. Non-limiting examples of these ammonium groups may include substituents such as hydroxypropyl trimmonium chloride, trimethylhydroxypropyl ammonium chloride, dimethylstearylhydroxypropyl ammonium chloride, and dimethyldodecylhydroxypropyl ammonium chloride. See Solarek, D. B., Cationic Starches in Modified Starches: Properties and Uses, Wurzburg, O.B., Ed., CRC Press, Inc., Boca Raton, Florida 1986, pp 113-125. The cationic groups may be added to the starch prior to degradation to a smaller molecular weight or the cationic groups may be added after such modification.

As used herein, the "degree of substitution" of the cationically modified starch polymers is an average measure of the number of hydroxyl groups on each anhydroglucose unit which is derivatized by substituent groups. Since each anhydroglucose unit has three potential hydroxyl groups available for substitution, the maximum possible degree of substitution is 3. The degree of substitution is expressed as the number of moles of substituent groups per mole of anhydroglucose unit, on a molar average basis. The degree of substitution may be determined using proton nuclear magnetic resonance spectroscopy ("1H NMR") methods well known in the art. Suitable 1H NMR techniques include those described in "Observation on NMR Spectra of Starches in Dimethyl Sulfoxide, Iodine-Complexing, and Solvating in Water-Dimethyl Sulfoxide", Qin-Ji Peng and Arthur S. Perlin, Carbohydrate Research, 160 (1987), 57-72; and "An Approach to the Structural Analysis of Oligosaccharides by NMR Spectroscopy", J. Howard Bradbury and J. Grant Collins, Carbohydrate Research, 71, (1979), 15-25.

The cationically modified starch polymer may comprise maltodextrin. Thus, in one embodiment of the present invention, the cationically modified starch polymers may be further characterized by a Dextrose Equivalance ("DE") value of less than about 35, and more preferably from about 1 to about 20. The DE value is a measure of the reducing equivalence of the hydrolyzed starch referenced to dextrose and expressed as a percent (on dry basis). Starch completely hydrolyzed to dextrose has a DE value of 100, and unhydrolyzed starch has a DE value of 0. A suitable assay for DE value includes one described in "Dextrose Equivalent", Standard Analytical Methods of the Member Companies of the Corn Industries Research Foundation, 1st ed., Method E-26. Additionally, the cationically modified starch polymers of the present invention may comprise a dextrin. Dextrin is typically a pyrolysis product of starch with a wide range of molecular weights.

The source of starch before chemical modification can be chosen from a variety of sources such as tubers, legumes, cereal, and grains. Non-limiting examples of this source starch may include corn starch, wheat starch, rice starch, waxy corn starch, oat starch, cassava starch, waxy barley, waxy rice starch, glutenous rice starch, sweet rice starch, amioca, potato starch, tapioca starch, oat starch, sago starch, sweet rice, or mixtures thereof. Waxy corn starch is preferred.

In one embodiment, cationically modified starch polymers are selected from degraded cationic maize starch, cationic tapioca, cationic potato starch, and mixtures thereof. In another embodiment, cationically modified starch polymers are cationic corn starch

The starch, prior to degradation or after modification to a smaller molecular weight, may comprise one or more additional modifications. For example, these modifications may include cross-linking, stabilization reactions, phophorylations, and hydrolyzations. Stabilization reactions may include alkylation and esterification.

The cationically modified starch polymers may be incorporated into the composition in the form of hydrolyzed starch (e.g., acid, enzyme, or alkaline degradation), oxidized starch (e.g., peroxide, peracid, hypochlorite, alkaline, or any other oxidizing agent), physically/mechanically degraded starch (e.g., via the thermo-mechanical energy input of the processing equipment), or combinations thereof.

Also suitable for use in the present invention is nonionic modified starch that could be further derivatized to a cationically modified starch as is known in the art. Other suitable modified starch starting materials may be quaternized, as is known in the art, to produce the cationically modified starch polymer suitable for use in the invention.

**Starch Degradation Procedure**

In one embodiment, a starch slurry is prepared by mixing granular starch in water. The temperature is raised to about 35°C. An aqueous solution of potassium permanganate is then added at a concentration of about 50 ppm based on starch. The pH is raised to about 11.5 with sodium hydroxide and the slurry is stirred sufficiently to prevent settling of the starch. Then, about a 30% solution of hydrogen peroxide diluted in water is added to a level of about 1 % of peroxide based on starch. The pH of about 11.5 is then restored by adding additional sodium hydroxide. The reaction is completed over about a 1 to about 20 hour period. The mixture is then neutralized with dilute hydrochloric acid. The degraded starch is recovered by filtration followed by washing and drying.

### Galactomannan Polymer Derivative

The personal care compositions of the present invention may comprise a galactomannan polymer derivative having a mannose to galactose ratio of greater than 2 : 1 on a monomer to monomer basis, the galactomannan polymer derivative is selected from the group consisting of a cationic galactomannan polymer derivative and an amphoteric galactomannan polymer derivative having a net positive charge. The term "galactomannan polymer derivative", means a compound obtained from a galactomannan polymer (*ie*. a galactomannan gum). As used herein, the term "cationic galactomannan" refers to a galactomannan polymer to which a cationic group is added. The term "amphoteric galactomannan" refers to a galactomannan polymer to which a cationic group and an anionic group are added such that the polymer has a net positive charge.

The gum for use in preparing the non-guar galactomannan polymer derivatives is typically obtained as naturally occurring material such as seeds or beans from plants. Examples of various non-guar galactomannan polymers include but are not limited to Tara gum (3 parts mannose / 1 part galactose), Locust bean or Carob (4 parts mannose / 1 part galactose), and Cassia gum (5 parts mannose / 1 part galactose). Herein, the term "non-guar galactomannan polymer derivatives" refers to cationic polymers which are chemically modified from a non-guar galactomanan polymer. A preferred non-guar galactomannan polymer derivative is cationic cassia, which is sold under the trade name, Cassia EX-906, and is commercially available from Noveon Inc.

The galactomannan polymer derivatives have a molecular weight of from about 1,000 to about 10,000,000. In one embodiment, the galactomannan polymer derivatives have a molecular weight of from about 5,000 to about 3,000,000. As used herein, the term "molecular weight" refers to the weight average molecular weight. The weight average molecular weight may be measured by gel permeation chromatography.

The personal care compositions may comprise at least about 0.05% of a galactomannan polymer derivative by weight of the composition. In one embodiment, the personal care compositions comprise from about 0.05% to about 2 %, by weight of the composition, of a galactomannan polymer derivative. Suitable galactomannan polymer derivatives are described in U.S. Patent Publication No. 2006/0099167A1 to Staudigel et al.

### Aqueous Carrier

The personal care compositions comprise an aqueous carrier which is generally present at a level of from about 20% to about 95%, more preferably from about 60% to about 85%. The aqueous carrier may comprise water, or a miscible mixture of water and organic solvent, but preferably comprises water with minimal or no significant concentrations of organic solvent, except as otherwise incidentally incorporated into the composition as minor ingredients of other essential or optional components.

### Non-amino-functionalized Silicone (NAFS)

In embodiments containing NAFS, the weight ratio of aminosilicone to NAFS is preferably from about 1:2 to about 1:99.9, more preferably from about 1:5 to about 1:99, more preferably 5:95. Preferably the NAFS has a viscosity of at least about 10,000cs, more preferably from about 60,000cs to about 2,000,000cs more preferably from about 100,000cs to about 500,000cs.

The NAFS component may comprise volatile NAFS, non-volatile NAFS, or combinations thereof. Preferred are non-volatile NAFS. If volatile NAFS are present, it will typically be incidental to their use as a solvent or carrier for commercially available forms of non-volatile NAFS materials ingredients, such as NAFS gums and resins. The NAFS may comprise a silicone fluid conditioning agent and may also comprise other ingredients, such as a NAFS resin to improve silicone fluid deposition efficiency or enhance glossiness of the hair.

The concentration of NAFS typically ranges from about 0.01% to about 10%, preferably from about 0.1 % to about 8%, more preferably from about 0.1 % to about 5%, more preferably from about 0.2% to about 3%. Non-limiting examples of suitable NAFS, and optional suspending agents for the silicone, are described in U.S. Reissue Patent No. 34,584, U.S. Pat. No. 5,104,646, and U.S. Pat. No. 5,106,609.

Background material on silicones including sections discussing silicone fluids, gums, and resins, as well as manufacture of silicones, are found in Encyclopedia of Polymer Science and Engineering, vol. 15, 2d ed., pp 204 308, John Wiley & Sons, Inc. (1989).

Embodiments containing a blend of aminosilicone and NAFS provide several benefits, including improved deposition of the silicone component and improved hair feel over compositions containing NAFS as the sole silicone component. Additionally, as aminosilicones are generally more expensive than NAFS, compositions containing both materials will generally be less expensive than those containing only aminosilicone as the silicone component, yet still provide improved hair conditioning versus compositions containing NAFS as the sole silicone component. Suitable NAFS materials are described in U.S. Patent Publication No. 2006/0127345A1.

**Dispersed Gel Network Phase**

The personal care compositions of the present invention may also comprise a dispersed gel network phase comprising a fatty amphiphile. The gel network phase may be included in personal care compositions of the present invention to provide conditioning benefits. As used herein, the term "gel network" refers to a lamellar or vesicular solid crystalline phase which comprises at least one fatty amphiphile as specified below, at least one secondary surfactant as specified below, and water or other suitable solvents. The lamellar or vesicular phase comprises bi-layers made up of a first layer comprising the fatty amphiphile and the secondary surfactant and alternating with a second layer comprising the water or other suitable solvent. The term "solid crystalline", as used herein, refers to the structure of the lamellar or vesicular phase which forms at a temperature below the melt transition temperature (i.e., the chain melt temperature) of the layer in the gel network comprising the one or more fatty amphiphiles, the melt transition temperature being at least about 27 °C. The melt transition temperature may be measured by differential scanning calorimetry, a method of which is described in the Examples below.

Gel networks, generally, are further described by G.M. Eccleston, "Functions of Mixed Emulsifiers and Emulsifying Waxes in Dermatological Lotions and Creams", Colloids and Surfaces A: Physiochem. and Eng. Aspects 123-124 (1997) 169-182; and by G.M Eccleston, "The Microstructure of Semisolid Creams", Pharmacy International, Vol. 7, 63-70 (1986).

In an embodiment of the present invention, the dispersed gel network phase is pre-formed. The term "pre-formed", as used herein, means that at least fifty percent of the mixture of the fatty amphiphile, secondary surfactant, and water or other suitable solvent is substantially a solid crystalline phase when added to the other components of the personal care composition.

According to this embodiment, the gel network component of the present invention is prepared as a separate pre-mix, which, after being cooled, is subsequently incorporated with the detersive surfactant and the other components of the personal care composition. Preparation of the gel network component is discussed in detail in U.S. Patent Publication No. 2006/0024256 A1.

The cooled and pre-formed gel network component subsequently is added to the other components of the personal care composition, including the detersive surfactant component. While not intending to be limited by theory, it is believed that incorporation of the cooled and pre-formed gel network component with the detersive surfactant and other components of the personal care composition allows the formation of a substantially equilibrated lamellar dispersion ("ELD") in the final personal care composition. The ELD is a dispersed lamellar or vesicular phase resulting from the pre-formed gel network component substantially equilibrating with the detersive surfactants, water, and other optional components, such as salts, which may be present in the personal care composition. This equilibration occurs upon incorporation of the pre-formed gel network component with the other components of the personal care composition and is effectively complete within about 24 hours after making. Personal care compositions in which the ELD is formed provide hair with improved wet and dry conditioning benefits. Further, the ELD does not form if the components which comprise the gel network component (i.e., the fatty amphiphile and the secondary surfactant combined with water) are added as individual components together with the other components of the personal care composition in one mixing step, and not as a separate cooled pre-formed gel network component.

As described above, the ELD is formed by the incorporation of the cooled and pre-formed gel network component with the detersive surfactant and other components of the personal care composition. While the ELD and the pre-formed gel network component both comprise fatty amphiphile, secondary surfactant, and water together in the form of a lamellar or vesicular solid crystalline phase, differences exist between certain physical properties of the ELD compared with those of the pre-formed gel network component. Prior to incorporation with the detersive surfactant and other components of the personal care composition, the pre-formed gel network component consists essentially of fatty amphiphile, secondary surfactant, and water. Upon incorporation, the lamellar structure of the gel network, acting as a template, is swelled by and equilibrates with the detersive surfactant and other components of the personal care composition, such as salts and perfumes. Thus, it is believed that these differences in certain physical properties between the pre-formed gel network component and the ELD are consistent with the migration of, for example, the detersive surfactant, salts, and perfumes, into the gel network phase.

The presence of the gel network in the pre-mix and in the final personal care composition in the form of the ELD can be confirmed by means known to one of skill in the art, such as X-ray analysis, optical microscopy, electron microscopy, and differential scanning calorimetry. Methods of X-ray analysis and differential scanning calorimetry are described in U.S. Patent Publication No. 2006/0024256 A1.

In one embodiment of the present invention, the scale size of the dispersed gel network phase in the personal care composition (i.e., the ELD) ranges from about 10 nm to about 500 nm. In another embodiment, the scale size of the dispersed gel network phase in the personal care composition ranges from about 0.5 $\mu$m to about 10 $\mu$m. In yet another embodiment, the scale size of the dispersed gel network phase in the personal care composition ranges from about 10 $\mu$m to about 150 $\mu$m.

The scale size distribution of the dispersed gel network phase in the personal care composition may be measured with a laser light scattering technique, using a Horiba model LA 910 Laser Scattering Particle Size Distribution Analyzer (Horiba Instruments, Inc. Irvine California, USA). The scale size distribution in a personal care composition of the present invention may be measured according to the method described in U.S. Patent Publication No. 2006/0269502A1.

In one embodiment, the personal care composition comprises a gel network in an amount greater than about 0.1 %, preferably from about 1 % to about 60 %, and more preferably from about 5. % to about 40 %, by weight of the personal care composition.

The following represent examples of the current invention where the particle size of the amino silicone is with 15 micron (μm):

| | Amino Examples | | | Non-Amino Test Controls | | |
|---|---|---|---|---|---|---|
| Ingredient | 1 | 2 | | A | B | C |
| Water | q.s. | q.s. | | q.s | q.s | q.s. |
| Cationic Galactomannan [1] | 0.25 | 0.25 | | -- | -- | -- |
| Cationic Starch[2] | -- | -- | | -- | -- | -- |
| Polyquaternium-10 (JR30M) | -- | -- | | 0.10 | -- | -- |
| Polyquaternium-10 (LR400) | -- | -- | | -- | 0.50 | -- |
| Guar 400M (0.7 CD) | -- | -- | | | -- | 0.50 |
| Sodium Laureth Sulfate (SLE3S -28% active) [3] | 28.57 | 28.57 | | 53.57 | -- | -- |
| Sodium Lauryl Sulfate (SLS - 29% active) [4] | 22.07 | 13.79 | | 17.24 | -- | -- |
| Ammonium Laureth Sulfate (ALE3S-25% active) | -- | -- | | -- | 40.00 | 40.00 |
| Ammonium Lauryl Sulfate (ALES-25% active) | -- | -- | | -- | 24.00 | 24.00 |
| Cocoamidopropyl Betain [5] | 7.00 | 6.67 | | -- | -- | -- |
| Pomidium 2 | -- | -- | | 2.00 | -- | -- |
| Cocamide MEA [6] | 0.50 | 0.50 | | -- | 0.80 | 0.80 |
| Cetyl Alcohol | -- | -- | | -- | 0.90 | 0.9 |
| PEG7M | -- | -- | | -- | 0.10 | -- |
| Ethylene Glycol Distearate [7] | 1.50 | 1.50 | | -- | 1.50 | 1.50 |
| Hydrogenated Polydecene | -- | -- | | | 0.30 | 0.40 |
| Trimethylolpropane Tricaprylate/Tricaprate | -- | -- | | | 0.10 | 0.10 |
| Fragrance | 0.70 | 0.70 | | 0.70 | 0.70 | 0.70 |
| Preservatives, pH adjusters | Up to 1% | Up to 1% | | Up to 1% | Up to 1% | Up to 1% |
| Sodium Chlorides [8] | 1.00 | 1.50 | | 1.50 | 1.50 | 1.50 |
| Aminosiloxane1 [9] | 0.50 | 1.00 | | -- | -- | -- |
| Dimethicone as active (premix made to 20-30 μm) [10] | -- | -- | | -- | 1.35 | 2.35 |
| Calculated: | | | | | | |
| Ethoxylate level | 2.35 | 2.35 | | 4.41 | 1.77 | 1.77 |

(continued)

| | Amino Examples | | | Non-Amino Test Controls | | |
|---|---|---|---|---|---|---|
| Ingredient | 1 | 2 | | A | B | C |
| Sulfate level | 3.21 | 2.57 | | 4.16 | 3.80 | 3.80 |

1 Cationic Galactomannan Cassia EX-906, MW = 300,000 CD = 3.1 meq/gram, Supplier Noveon Inc.
2 Cationic Starch MW = 8,000,000-10,000,000 CD= 3.2 meq/gram, Supplier National Starch
3 Sodium Laureth Sulfate at 28% active, supplier: P&G
4 Sodium Lauryl Sulfate at 29% active, supplier: P&G
5 Tegobetaine F-B, 30% active, supplier: Goldschmidt Chemicals
6 Monamid CMEA, supplier Goldschmidt Chemical
7 Ethylene Glycol Distearate EGDS Pure; Supplier Gold Schmidt Chemicals
8 Sodium Chloride USP (food grade), supplier Morton.
9 GE Silicones Aminosiloxane, GE Y-14945; Viscosity = 10,000 cps.
10 GE Silicones dimethicone Viscasil 330M, Viscosity = 330,000 cps.

Method of Making for products for 15 μm terminal amino silicone

Examples 1 and 2 above are made by mixing ingredient together in the order listed above. A critical point in formulation that is necessary in order to achieve a 15 μm particle size of the amino silicone is that an effective amount of the salt must be added to the formulation prior to the addition of the amino silicone in order to adjust to a viscosity of at least about 4,000cps. One skill in the art of making colloids can make the proper adjustments to meet this target particle size without undue experimentation. Another important aspect of the making procedure for 15 μm is to confirm that after the addition of the terminal amino silicone, mixing at a speed of at about 200 rpm for at least 15 minutes is performed on the formulation.

Demonstration of Conditioning Benefits

The efficient and superior conditioning benefits of the examples can be demonstrated by measurement of the friction of the dry hair surface. Lower friction values demonstrate superior dry conditioning. As shown in the data below, the shampoo samples containing lower levels of amino silicone and a high charge density cationic galactomannan show the lowest friction on hair versus products containing higher levels of standard non-amino silicones and lower charge density polymers.

**Dry Friction Data Test #1**

| Formula | Mean Friction |
|---|---|
| Example 1 (0.5% Amino silicone) | 88.53 |
| Control B (1.35% non-amino silicone) | 99.28 |
| Control A (no silicone) | 128.07 |

**Dry Friction Data Test #2**

| Formula | Mean Friction |
|---|---|
| Example 2 | 80.80 |
| Control C (2.35% non-amino silicone) | 93.39 |
| Control B (1.35% non-amino silicone) | 95.54 |
| Control C (no silicone) | 123.44 |

The following are additional examples of the current invention with an amino particle size approximately 15 microns.

| Ingredient | 3 | 4 | 5 | 6 | 7 | 8 | 9 | 10 |
|---|---|---|---|---|---|---|---|---|
| Water | q.s. | q.s. | q.s. | q.s. | q.s. | q.s. | q.s. | q.s. |
| Cationic Galactomannan [1] | 0.25 | 0..15 | 0.5 | 0.1 | -- | -- | -- | -- |
| Cationic Starch [2] | -- | -- | -- | -- | 0.25 | 0.10 | 0.10 | 0.25 |
| Sodium Laureth Sulfate (SLE3S -28% active) [3] | 28.57 | 28.57 | 35.71 | 53.57 | 28.57 | 28.57 | 28.57 | 28.57 |
| Sodium Lauryl Sulfate (SLS - 29% active) [4] | 22.07 | 13.79 | 6.90 | 0 | 22.07 | 13.79 | 22.07 | 13.79 |
| Cocoamidopropyl Betaine [5] | 7.00 | 6.67 | 6.67 | 16.67 | 7.00 | 6.67 | 7.00 | 6.67 |
| Cocamide MEA [6] | 0.5 | 0.5 | 0.5 | 0.5 | 0.5 | 0.5 | 0.5 | 0.5 |
| Ethylene Glycol Distearate [7] | 1.5 | 1.5 | 1.5 | 1.5 | 1.5 | 1.5 | 1.5 | 1.5 |
| Fragrance | 0.70 | 0.70 | 0.70 | 0.70 | 0.70 | 0.70 | 0.70 | 0.70 |
| Preservatives, pH adjusters | Up to 1% | Up to 1% | Up to 1% | Up to 1% | Up to 1% | Up to 1% | Up to 1% | Up to 1% |
| Sodium Chloride [8] | 1.0 | 1.5 | 1.0 | 1.0 | 1.0 | 1.5 | 1.0 | 1.5 |
| Aminosiloxane GE Y-14945 [9] | -- | -- | 0.5 | 0.5 | 0.5 | 1.0 | -- | -- |
| Aminosiloxane GE Y-14935 [10] | 0.5 | 1.0 | 0.5 | -- | -- | -- | 0.5 | 1.0 |
| Calculated: | | | | | | | | |
| Ethoxylate level | 2.35 | 2.35 | 2.94 | 4.41 | 2.35 | 2.35 | 2.35 | 2.35 |
| Sulfate level | 3.21 | 2.57 | 2.42 | 2.82 | 3.21 | 2.57 | 3.21 | 2.57 |

1 Cationic Galactomannan Cassia EX-906, MW = 300,000 CD = 3.1 meq/gram, Supplier Noveon Inc.
2 Cationic Starch MW = 8,000,000-10,000,000 CD= 3.2 meq/gram, Supplier National Starch
3 Sodium Laureth Sulfate at 28% active, supplier: P&G
4 Sodium Lauryl Sulfate at 29% active, supplier: P&G
5 Tegobetaine F-B, 30% active, supplier: Goldschmidt Chemicals
6 Monamid CMEA, supplier Goldschmidt Chemical
7 Ethylene Glycol Distearate EGDS Pure; Supplier Gold Schmidt Chemicals
8 Sodium Chloride USP (food grade), supplier Morton.
9 GE Silicones Aminosiloxane, GE Y-14945; Viscosity = 10,000 cps.
10 GE Silicones Aminosiloxane, GE Y-14935; Viscosity = 300,000 cps.

The same method of making aspects used for Examples 1 and 2 are used for Examples 3 through 10 and are also used with examples 11 through 15 below.

The following examples are representative of the use of amino silicone in combination with non-amino silicones.

| Ingredient | 11 | 12 | 13 |
|---|---|---|---|
| Water | q.s. | q.s. | q.s. |
| Catonic Galactomannan [1] | 0.25 | 0.25 | 0.25 |
| Sodium Laureth Sulfate (SLE3S -28% active) [2] | 28.57 | 28.57 | 28.57 |
| Sodium Lauryl Sulfate (SLS - 29% active) [3] | 22.07 | 22.07 | 22.07 |

(continued)

| Ingredient | 11 | 12 | 13 |
|---|---|---|---|
| Ammonium Laureth Sulfate (ALE3S-25% active) | -- | -- | -- |
| Ammonium Lauryl Sulfate (ALES-25% active) | -- | -- | -- |
| Cocoamidopropyl Betaine [4] | 7.00 | 7.00 | 7.00 |
| Pomidium 2 | -- | -- | -- |
| Cocamide META [5] | 0.50 | 0.50 | 0.50 |
| Cetyl Alcohol | -- | -- | -- |
| PEG7M | -- | -- | -- |
| Ethylene Glycol Distearate [6] | 1.50 | 1.50 | 1.50 |
| Mobil P43 (synthetic oil) | -- | -- | -- |
| Puresyn (synthetic oil) | -- | -- | -- |
| Fragrance | 0.70 | 0.70 | 0.70 |
| Preservatives, pH adjusters | Up to 1% | Up to 1 % | Up to 1 % |
| Sodium Chloride [7] | 1.00 | 1.00 | 1.00 |
| Aminosiloxane, Y-14945 [8] | 0.80 | 0.50 | 0.20 |
| Dimethicone as active (premix made to 20-30 μm)[9] | 0.20 | 0.50 | 0.80 |
| Ethoxylate level | 2.35 | 2.35 | 2.35 |
| Sulfate level | 3.21 | 3.21 | 3.21 |

1 Cationic Galactomannan Cassia EX-906 MW = 300,000 CD = 3.1 meq/gram, Supplier Noveon Inc.
2 Sodium Laureth Sulfate at 28% active, supplier: P&G
3 Sodium Lauryl Sulfate at 29% active, supplier: P&G
4 Tegobetaine F-B, 30% active, supplier: Goldschmidt Chemicals
5 Monamid CMEA, supplier Goldschmidt Chemical
6 Ethylene Glycol Distearate EGDS Pure; Supplier Gold Schmidt Chemicals
7 Sodium Chloride USP (food grade), supplier Morton.
8 GE Silicones Aminosiloxane GE Y-14945; Viscosity = 10,000 cps.
9 GE Silicones Dimethicone Fluid, Viscasil 330M (Viscosity = 330,000cps)

Examples with 15 um particle terminal amino silicone mixed with >20 um particle DC- 330M silicones

Upon mixing in terminal amino silicone one must mix at 200 rpm for 15 minutes to obtain approximate particle size of 15 um. Before adding DC-330M one should slow the speed to 100 rpm for 15 minutes to achieve particle size of >20 um.

Dry friction data Test #3

| Formula | Mean Friction |
|---|---|
| Example 11 | 83.41 |
| Example 12 | 85.13 |
| Example 13 | 89.31 |
| Control B (1.35% non-amino silicone | 99.28 |
| Control A (no silicone) | 128.07 |

The following are examples where dimethicone emulsions (<1 μm) are present with approximately 15 μm particle terminal amino silicone.

| Ingredient | 14 | 15 |
|---|---|---|
| Water | q.s. | q.s. |
| Catonic Galactomannan [1] | 0.25 | 0.25 |
| Sodium Laureth Sulfate (SLE3S -28% active) [2] | 28.57 | 28.57 |
| Sodium Lauryl Sulfate (SLS - 29% active) [3] | 13.79 | 13.79 |
| Cocoamidopropyl Betaine [4] | 6.67 | 6.67 |
| Cocamide MEA [5] | 0.50 | 0.50 |
| Ethylene Glycol Distearate [6] | 1.50 | 1.50 |
| Fragrance | 0.70 | 0.70 |
| Preservatives, pH adjusters | Up to 1% | Up to 1% |
| Sodium Chloride [7] | 1.00 | 1.00 |
| Aminosiloxane, Y-14945 [8] | 1.00 | 0.20 |
| Dimethiconol Microemulsion [9] | 0.20 | -- |
| Dimethicone emulsion [10] | -- | 1.00 |
| Calculated: | | |
| Ethoxylate level | 2.35 | 2.35 |
| Sulfate level | 2.57 | 2.57 |

1 Cationic Galactomannan Cassia EX-906 MW = 300,000 CD =3.1 meq/gram, Supplier Noveon Inc.
2 Sodium Laureth Sulfate at 28% active, supplier: P&G
3 Sodium Lauryl Sulfate at 29% active, supplier: P&G
4 Tegobetaine F-B, 30% active, supplier: Goldschmidt Chemicals
5 Monamid CMEA, supplier Goldschmidt Chemical
6 Ethylene Glycol Distearate EGDS Pure; Supplier Gold Schmidt Chemicals
7 Sodium Chloride USP (food grade), supplier Morton.
8 GE Silicones Aminosiloxane GE Y-14945; Viscosity = 10,000 cps.
9 DC2-1865, Internal phase viscosity = 25,000cps, 25nm particle size size dimethiconol. Use TEA dodecyl benzene sulfonate and laureth 23 as primary surfactants
10 Dow Corning Dimethicone emulsion DC-1664; 300 nm particle size

Examples < 1um dimethicone microemulsion mixed with 15 um particle terminal amino silicone

One should mix in the terminal amino silicone at 200 rpm for 15 minutes. Then one should mix in dimethicone microemulsion at 100 rpm for 15 minutes.

The following are examples of the present invention where amino silicones are < 1 $\mu$n.

| Ingredient | 16 | 17 | 18 | 19 | 20 | 21 | 22 |
|---|---|---|---|---|---|---|---|
| Water | q.s. | q.s. | q.s. | q.s. | q.s. | q.s. | q.s. |
| Catonic Galactomannan [1] | 0.25 | 0.25 | 0.25 | 0.25 | 0.25 | 0.25 | 0.25 |
| Sodium Laureth Sulfate (SLE3S -28% active) [2] | 28.57 | 28.57 | 28.57 | 35.71 | 53.57 | 28.57 | 28.57 |
| Sodium Lauryl Sulfate (SLS - 29% active) [3] | 22.07 | 22.07 | 22.07 | 6.90 | 0 | 22.07 | 22.07 |
| Cocoamidopropyl Betaine [4] | 7.00 | 7.00 | 7.00 | 6.67 | 16.67 | 7.00 | 7.00 |
| Cocamide MEA [5] | 0.50 | 0.50 | 0.50 | 0.50 | 0.50 | 0.50 | 0.50 |
| Ethylene Glycol Distearate [6] | 1.50 | 1.50 | 1.50 | 1.50 | 1.50 | 1.50 | 1.50 |

(continued)

| Ingredient | 16 | 17 | 18 | 19 | 20 | 21 | 22 |
|---|---|---|---|---|---|---|---|
| Fragrance | 0.70 | 0.70 | 0.70 | 0.70 | 0.70 | 0.70 | 0.70 |
| Preservatives, pH adjusters | Up to 1% | Up to 1% | Up to 1% | Up to 1% | Up to 1% | Up to 1% | Up to 1% |
| Amodimethicone [7] | 0.50 | -- | -- | 0.50 | 0.50 | -- | -- |
| Amodimethicone [8] | -- | 0.50 | -- | -- | -- | -- | -- |
| Amodimethicone [9] | -- | -- | 0.50 | -- | -- | -- | -- |
| Aminosiloxane [10] | -- | -- | -- | -- | -- | 2.0 | -- |
| Amodimethicone (microemulsion) [11] | -- | -- | -- | -- | -- | -- | 2.0 |
| Sodium Chloride | 1.00 | 1.00 | 1.00 | 1.00 | 1.00 | 1.00 | 1.00 |
| Ethoxylate level | 2.35 | 2.35 | 2.35 | 2.94 | 4.41 | 2.35 | 2.35 |
| Sulfate level | 3.21 | 3.21 | 3.21 | 2.42 | 2.82 | 3.21 | 3.21 |

1 Cationic Galactomannan Cassia Ex-906, MW= 300,000 CD=3.1 meq/gram, supplier Noveon Inc.
2 Sodium Laureth Sulfate at 28% active, supplier: P&G
3 Sodium Lauryl Sulfate at 29% active, supplier: P&G
4 Tegobetaine F-B, 30% active, supplier: Goldschmidt Chemicals
5 Monamid CMEA, supplier Goldschmidt Chemical
6 Ethylene Glycol Distearate EGDS Pure; Supplier Gold Schmidt Chemicals
7 Dow Corning Amodimethicone DC-8500, Viscosity=4,000 cts; 100% Active
8 Dow Corning Amodimethicone DC-8566, Viscosity=3,500 cts; 100% Active
9 GE Silicones Amodimethicone SF-1708, Viscosity 2,500 cts; 100% Active
10 GE Silicones Aminosiloxane GE-253, Viscosity 2,000cts; 20% active
12 Wacker Silicones Amodimethicone (microemulsion) ADM 8020 VP, Viscosity <50 [$mm^2$/s], 15% Active
13 Sodium Chloride USP (food grade), supplier Morton.

Examples with < 1 um particle size amino silicones

In order to achieve small particle size amino silicones, salt adjustments should be made after the introduction of these materials. A lower viscosity of the bulk formulation will help aid in particle size reduction. It is important to mix in amino silicones at a speed of at least 200 rpm for at least 15 minutes. Then one may make the salt additions to adjust to final viscosity targets.

The following Examples illustrate specific embodiments of the gel network pre-mix, prior to its incorporation with the detersive surfactant and other components of the final shampoo composition. It is intended that each of the following gel network pre-mix examples could be incorporated as a dispersed phase into a shampoo composition according to the present invention.

| Ingredient | A |
|---|---|
| Water | 82.76% |
| Cetyl Alcohol | 3.00% |
| Cocamine oxide | |
| Glyceryl distearate (1) | |
| Sorbitan tristearate (1) | |
| Steary Alcohol | 5.57% |
| Stearamide MEA-stearate (1) | |
| Steareth-2, Volpa S-2 (2) | |
| Stearic acid, V-1890 (3) | |

(continued)

| Ingredient | A |
|---|---|
| Sucrose distearate, Crodesta F-10 (2) | |
| Sodium laureth-3 sulfate (28% Active) | 8.64% |
| 5-Chloro-2-methyl-4-isothiazolin-3-one, Kathon CG | 0.03% |

| Ingredient | B | C |
|---|---|---|
| Water | 88.55% | 88.55% |
| Stearyl Alcohol | | |
| Cetyl Alcohol | | |
| Glyceryl hydroxystearate (1) | | |
| PEG-2 Stearate (1) | | |
| Palmitic Acid | 3.00% | 5.72% |
| Steareth-2, Volpo S-2 (2) | | |
| Stearic acid, V-1890 (3) | 5.57% | 2.86% |
| Behenyltrimethylammonium chloride, Varisoft BT-85 (2) | 2.85% | 2.84% |
| 5-Chloro-2-methyl-4-isothiazolin-3-one, Kathon CG | 0.03% | 0.03% |
| (1) available from A&E Connock<br>(2) available from Croda Chemicals<br>(3) available from P&G Chemicals<br>(4) available Goldschmidt Chemical | | |

Preparation of Final Shampoo Compositions

In one embodiment, to prepare the final shampoo composition, first, a surfactant solution pre-mix is formed. To prepare this surfactant solution pre-mix, about 6% to about 9% of sodium or ammonium laureth-3 sulfate, cationic polymers, and about 0% to about 5% of water are added to a jacketed mix tank and heated to about 74°C with agitation. To this solution, citric acid, sodium citrate, sodium benzoate, and disodium EDTA are added to the tank and allowed to disperse. Ethylene glycol distearate (EGDS) is then added to the mixing vessel and melted. After the EGDS is well dispersed (e.g., after about 10 minutes), preservative is added and mixed into the surfactant solution. This mixture is passed through a mill and heat exchanger where it is cooled to about 35°C and collected in a finishing tank. As a result of this cooling step, the EGDS crystallizes to form a waxy crystalline suspension. The mixture of these components is the surfactant solution pre-mix.

Next, the surfactant solution pre-mix and the gel network pre-mix, which is prepared as described above, are mixed together. The remainder of the surfactants, perfume, dimethicone, sodium chloride or ammonium xylene sulfonate for viscosity adjustment, and the remainder of the water are added with ample agitation to ensure a homogeneous mixture. This mixture is the final shampoo composition which comprises as a dispersed phase the gel network pre-mix.

Preferred viscosities of the final shampoo composition according to the present invention range from about 5000 to about 15,000 centipoise at 27°C, as measured by a Wells-Brookfield model RVTDCP viscometer using a CP-41 cone and plate at 2/s at 3 minutes.

The pH may be adjusted as necessary to provide shampoo compositions of the present invention which are suitable for application to human hair, and may vary based on the selection of particular detersive surfactants, fatty amphiphiles, and/or other components.

Shampoo Examples

The following Examples illustrate specific embodiments of the final shampoo composition of the present invention, which respectively comprise select above-exemplified gel network premixes as a dispersed phase.

| Ingredient | 31 | 32 | 33 | 34 | 35 | 36 | 37 | 38 | 39 | 40 |
|---|---|---|---|---|---|---|---|---|---|---|
| Sodium Laureth Sulfate | 10.00 | 10.00 | 10.00 | 10.00 | 10.00 | 10.00 | 10.00 | 6.00 | 15.00 | 8.5 |
| Sodium Lauryl Sulfate | 1.50 | 1.50 | 1.50 | 1.50 | 1.50 | 1.50 | 6.00 | 10.00 | 5.00 | 3.00 |
| Cocamidopropyl betaine | 2.00 | 2.00 | 2.00 | 2.00 | 2.00 | 2.00 | 2.00 | 2.00 | 2.00 | 2.00 |
| Any Gel Network Composition | 27.3 | 13.6 | | | | | 27.3 | 27.3 | 27.3 | 27.3 |
| Gel Network A | | | 27.3 | 27.3 | | | | | | |
| Gel Network B | | | | | 27.3 | | | | | |
| Gel Network C | | | | | | 27.3 | | | | |
| Cationic Galactomannan (1) | 0.4 | 0.4 | 0.4 | 0.4 | | | 0.3 | 0.3 | 0.2 | 0.2 |
| Cationic Galactomannan (2) | | | | | 0.1 | 0.1 | | | 0.2 | |
| Guar Hydroxypropyl trimonium chloride (3) | | | | | | | | 0.1 | | |
| Guar Hydroxypropyl trimonium chloride (4) | | | | | 0.3 | 0.3 | | | | |
| Polyquaterium-10 (5) | | | | | | | 0.1 | | | |
| Dimethicone (6) | | | | 0.5 | 0.25 | 2.00 | | | | |
| Amiosiloxane (7) | 0.5 | 0.5 | | 0.5 | 1.0 | 0.25 | 1.00 | | 0.8 | 0.25 |
| Aminosiloxane (8) | | | 0.5 | | | | | 0.8 | | 0.25 |
| Ethylene Glycol Distearate | 1.5 | 1.5 | 1.5 | 1.5 | 3.0 | 1.5 | 1.5 | 1.5 | 1.5 | 1.5 |
| 5-Chloro-2-methyl-4-isothiazolin-3-one, Kathon CG | 0.000 5 | 0.0005 | 0.0005 | 0.0005 | 0.0005 | 0.0005 | 0.0005 | 0.0005 | 0.0005 | 0.0005 |
| Sodium Benzoate | 0.25 | 0.25 | 0.25 | 0.25 | 0.25 | 0.25 | 0.25 | 0.25 | 0.25 | 0.25 |
| Disodium EDTA | 0.13 | 0.13 | 0.13 | 0.13 | 0.13 | 0.13 | 0.13 | 0.13 | 0.13 | 0.13 |
| Perfume | 0.70 | 0.70 | 0.70 | 0.70 | 0.70 | 0.70 | 0.70 | 0.70 | 0.70 | 0.70 |
| Citric Acid/ Sodium Citrate Dihydrate | pH QS | pH QS | pH QS | pH QS | pH QS | pH QS | pH QS | pH QS | pH QS | pH QS |
| Sodium Chloride/ Ammonium Xylene Sulfonate | Visc. QS | Visc. QS | Visc. QS | Visc. QS | Visc. QS | Visc. QS | Visc. QS | Visc. QS | Visc. QS | Visc. QS |

(continued)

| Ingredient | 31 | 32 | 33 | 34 | 35 | 36 | 37 | 38 | 39 | 40 |
|---|---|---|---|---|---|---|---|---|---|---|
| Water | QS | QS | QS | QS | QS | QS | QS | QS | QS | QS |

(1) Cationic Galactomannan (with Mol. W. of ~ 200,000; Char. Den.= 3.0 meq/g)
(2) Cationic Galactomannan (with Mol. W. of ~ 200,000; Char. Den.= 0.7 meq/g)
(3) Jaguar C17 available from Rhodia
(4) ADPP-5043HMW (with Mol.W. of ~1,200,000 and Char.Den. of 2.0meq/g) available from Aqualon/Hercules
(5) Polymer LR30M available from Amerchol/Dow Chemical
(6) Viscasil 330M available from General Electric Silicones
(7) GE Y-14945 available from General Electric Silicones (10,000 cst terminal amino silicone)
(8) GE Y-14935 available from General Electric Silicones (300,000 cst terminal amino silicone)

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

All documents cited in the Detailed Description of the Invention are, in relevant part, incorporated herein by reference. To the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the scope of the invention.

## Claims

1. A personal care composition comprising:

    a.) from 5 wt.% to 50 wt.% of an anionic surfactant system, said anionic surfactant system comprising at least one anionic surfactant and having an ethoxylate level and an anion level,

        i) wherein said ethoxylate level is from 1.5 to 6, and
        ii) wherein said anion level is from 1.5 to 6;

    b.) a water insoluble aminosilicone, wherein said aminosilicone has an average particle size in the composition of less than or equal to 30 μm;
    c.) from 0.05 wt.% to 5 wt.% of a naturally derived cationic polymer derivative wherein said polymer derivative has a molecular weight from 1,000 to 10,000,000, and wherein said polymer derivative has a cationic charge density at least 3.0 meq/g.; and
    d.) aqueous carrier,

    said composition being **characterized in that** said naturally derived cationic polymer derivative comprises a non-guar galactomannan polymer derivative that is cationic cassia.

2. A composition according to Claim 1, wherein said naturally derived cationic polymer is present in an amount of from 0.05 wt.% to 2 wt.%.

3. A composition according to claim 1 or claim 2, wherein the naturally derived cationic polymer has an average charge density of from greater than 3.0 meq/g to 7 meq/g.

4. A composition according to any one of claims 1 to 3, wherein said aminosilicone has an average particle size in the composition of less than 1 μm.

5. A composition according to any one of claims 1 to 3, wherein said aminosilicone has an average particle size in the composition of from 1 to 30 μm.

6. A composition according to any one of the preceding Claims, wherein said aminosilicone is selected from the group consisting of terminal aminosilicone, grafted aminosilicones, and mixtures thereof, and said aminosilicone has a viscosity of from 5,000 cs to 500,000 cs.

7. A composition according to any one of the preceding Claims, further comprising a dispersed gel network phase.

8. A composition according any one of the preceding Claims, further comprising a non-amino-functionalized silicone.

## Patentansprüche

1. Körperpflegezusammensetzung, umfassend:

a.) 5 Gew.-% bis 50 Gew.-% eines anionisches Tensidsystems, wobei das anionische Tensidsystem mindestens ein anionisches Tensid umfasst und eine Ethoxylatzahl und eine Anionenzahl aufweist,

i) wobei die Ethoxylatzahl 1,5 bis 6 ist und
ii) wobei die Anionenzahl 1,5 bis 6 ist;

b.) ein wasserunlösliches Aminosilikon, wobei das Aminosilikon in der Zusammensetzung eine durchschnittliche Teilchengröße von weniger oder gleich 30 μm aufweist;
c.) 0,05 Gew.-% bis 5 Gew.-% eines natürlich abgeleiteten kationischen Polymerderivats, wobei das Polymerderivat ein Molekulargewicht von 1.000 bis 10.000.000 aufweist und wobei das Polymerderivat eine kationische Ladungsdichte von mindestens 3,0 Milliäquivalent/g aufweist; und
d.) einen wässrigen Träger,

wobei die Zusammensetzung **dadurch gekennzeichnet ist, dass** das natürlich abgeleitete kationische Polymerderivat ein Nicht-Guar-Galactomannan-Polymerderivat umfasst, das kationische Kassie ist.

**2.** Zusammensetzung nach Anspruch 1, wobei das natürlich abgeleitete kationische Polymer in einer Menge von 0,05 Gew.-% bis 2 Gew.-% vorhanden ist.

**3.** Zusammensetzung nach Anspruch 1 oder 2, wobei das natürlich abgeleitete kationische Polymer eine durchschnittliche Ladungsdichte von mehr als 3,0 Milliäquivalent/g bis 7 Milliäquivalent/g aufweist.

**4.** Zusammensetzung nach einem der Ansprüche 1 bis 3, wobei das Aminosilikon in der Zusammensetzung eine durchschnittliche Teilchengröße von weniger als 1 μm aufweist.

**5.** Zusammensetzung nach einem der Ansprüche 1 bis 3, wobei das Aminosilikon in der Zusammensetzung eine durchschnittliche Teilchengröße von 1 μm bis 30 μm aufweist.

**6.** Zusammensetzung nach einem der vorstehenden Ansprüche, wobei das Aminosilikon ausgewählt ist aus der Gruppe, bestehend aus endständigem Aminosilikon, gepfropften Aminosilikonen und Mischungen davon, und das Aminosilikon eine Viskosität im Bereich von 5.000 $mm^2/s$ bis 500.000 $mm^2/s$ (5.000 cSt bis 500.000 cSt) aufweist.

**7.** Zusammensetzung nach einem der vorstehenden Ansprüche, ferner eine dispergierte Gelnetzwerkphase umfassend.

**8.** Zusammensetzung nach einem der vorstehenden Ansprüche, ferner ein nichtaminofunktionalisiertes Silikon umfassend.

## Revendications

**1.** Composition de soin personnel comprenant :

a.) de 5 % en poids à 50 % en poids d'un système tensioactif anionique, ledit système tensioactif anionique comprenant au moins un agent tensioactif anionique et possédant un taux d'éthoxylate et un taux d'anions,

i) dans laquelle ledit taux d'éthoxylate va de 1,5 à 6, et
ii) dans laquelle ledit taux d'anions va de 1,5 à 6 ;

b.) une aminosilicone insoluble dans l'eau, où ladite aminosilicone présente une taille de particule moyenne dans la composition inférieure ou égale à 30 μm ;
c.) de 0,05 % en poids à 5 % en poids d'un dérivé de polymère cationique dérivé naturellement dans laquelle ledit dérivé de polymère a une masse moléculaire de 1000 à 10 000 000, et dans laquelle ledit dérivé de polymère présente une densité de charge cationique d'au moins 3,0 méq/g ; et
d.) un véhicule aqueux,

ladite composition étant **caractérisée en ce que** ledit dérivé de polymère cationique dérivé naturellement comprend un dérivé de polymère de galactomannane autre que gomme de guar qui est de la casse cationique.

**2.** Composition selon la revendication 1, dans laquelle ledit polymère cationique dérivé naturellement est présent en une quantité allant de 0,05 % en poids à 2 % en poids.

**3.** Composition de soin personnel selon la revendication 1 ou la revendication 2, dans laquelle le polymère cationique dérivé naturellement a une densité de charge cationique allant de plus de 3,0 méq/g à 7 méq/g.

**4.** Composition selon l'une quelconque des revendications 1 à 3, dans laquelle ladite aminosilicone présente une taille de particule moyenne dans la composition, inférieure à 1 $\mu$m.

**5.** Composition selon l'une quelconque des revendications 1 à 3, dans laquelle ladite aminosilicone possède une taille de particule moyenne dans la composition allant de 1 à 30 $\mu$m.

**6.** Composition selon l'une quelconque des revendications précédentes, dans laquelle ladite aminosilicone est choisie parmi le groupe constitué d'une aminosilicone terminale, d'aminosilicones greffées et de mélanges de celles-ci, et ladite aminosilicone présente une viscosité allant de 5000 $mm^2/s$ à 500 000 $mm^2/s$ (5000 cs à 500 000 cs).

**7.** Composition selon l'une quelconque des revendications précédentes, comprenant en outre une phase de réseau de gel dispersée.

**8.** Composition selon l'une quelconque des revendications précédentes, comprenant en outre une silicone sans fonction amine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030224954 A, (Wells
- US 20060127345 A, Hilvert
- US 5104646 A, Bolich Jr.
- US 5106609 A, Bolich Jr.
- US 4152416 A, Spitzer
- US 2891920 A, Hyde
- US 3294725 A, Findlay
- US 3360491 A, Axon
- US 20060099167 A1, Staudigel
- US 34584 A
- US 20060127345 A1
- US 20060024256 A1
- US 20060269502 A1


### Non-patent literature cited in the description


- **J. CAELLES et al.** Anionic and Cationic Compounds in Mixed Systems. *Cosmetics & Toiletries,* April 1991, vol. 106, 49-54
- **C. J. VAN OSS.** Coacervation, Complex Coacervation and Flocculation. *J. Dispersion Science and Technology,* 1988, vol. 9 (5,6), 561-573
- **D. J. BURGESS.** Practical Analysis of Complex Coacervate Systems. *J. of Colloid and Interface Science,* November 1990, vol. 140 (1), 227-238
- **SOLAREK, D. B.** Cationic Starches in Modified Starches: Properties and Uses. CRC Press, Inc, 1986, 113-125
- **QIN-JI PENG ; ARTHUR S. PERLIN.** Observation on NMR Spectra of Starches in Dimethyl Sulfoxide, Iodine-Complexing, and Solvating in Water-Dimethyl Sulfoxide. *Carbohydrate Research,* 1987, vol. 160, 57-72
- **J. HOWARD BRADBURY ; J. GRANT COLLINS.** An Approach to the Structural Analysis of Oligosaccharides by NMR Spectroscopy. *Carbohydrate Research,* 1979, vol. 71, 15-25
- Encyclopedia of Polymer Science and Engineering. John Wiley & Sons, Inc, 1989, vol. 15, 204-308
- **G.M. ECCLESTON.** Functions of Mixed Emulsifiers and Emulsifying Waxes in Dermatological Lotions and Creams. *Colloids and Surfaces A: Physiochem. and Eng. Aspects,* 1997, vol. 123-124, 169-182
- **G.M ECCLESTON.** The Microstructure of Semisolid Creams. *Pharmacy International,* 1986, vol. 7, 63-70